(19) Europäisches Patentamt
European Patent Office
Office européen des brevets

(11) EP 3 305 897 A1

(12) # EUROPEAN PATENT APPLICATION
published in accordance with Art. 153(4) EPC

(43) Date of publication:
**11.04.2018 Bulletin 2018/15**

(21) Application number: **16803206.8**

(22) Date of filing: **26.05.2016**

(51) Int Cl.:
***C12N 15/09*** *(2006.01)* ***C12N 1/21*** *(2006.01)*
***C12P 7/62*** *(2006.01)*

(86) International application number:
**PCT/JP2016/065587**

(87) International publication number:
**WO 2016/194771 (08.12.2016 Gazette 2016/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.05.2015 JP 2015110777**

(71) Applicant: **National University Corporation Hokkaido University**
**Sapporo-shi, Hokkaido 060-0808 (JP)**

(72) Inventors:
- **TAGUCHI, Seiichi**
  **Sapporo-shi**
  **Hokkaido 060-0808 (JP)**
- **KADOYA, Ryosuke**
  **Sapporo-shi**
  **Hokkaido 060-0808 (JP)**

(74) Representative: **Zwicker, Jörk**
**ZSP Patentanwälte PartG mbB**
**Hansastraße 32**
**80686 München (DE)**

(54) **mtgA GENE-DEFICIENT MICROORGANISM**

(57) Provided is a bacterial strain having a mutation in the gene that codes for monofunctional peptidoglycan transglycosylase (MtgA), wherein the mutation inactivates MtgA or reduces the activity of MtgA in comparison to a control strain lacking the mutation.

EP 3 305 897 A1

SEQUENCE LISTING

<110> NATIONAL UNIVERSITY CORPORATION HOKKAIDO UNIVERSITY

<120> mtgA gene deletion microorganism

<130> 672898

<150> JP2015-110777
<151> 2015-05-29

<160> 3

<170> PatentIn version 3.5

<210> 1
<211> 20
<212> DNA
<213> Artificial

<220>
<223> primer

<400> 1
aaggtgatcc ggtggatgac 20

<210> 2
<211> 22
<212> DNA
<213> Artificial

<220>
<223> primer

<400> 2
caatcggctg ctctgatgcc gc 22

<210> 3
<211> 729
<212> DNA
<213> Escherichia coli

<400> 3
atgagtaaaa gccgcttaac ggtgtttagt ttcgttcgcc gttttctttt gcggttaatg 60

gttgtcctcg ccgttttctg ggcgggggc atcgcgttgt ttagcgttgc gcctgttccc 120

ttctcagcgg taatggtcga gcgacaggtc agcgcctggc tgcatggcaa ttttcgttac 180

gtggcacatt ctgactgggt cagtatggat caaatctcgc cgtggatggg actggcggtg 240

attgccgcag aagatcagaa atttcctgag cactggggct ttgatgtcgc ttccattgag 300

aaagccctgg cgcacaacga gcgcaatgaa aaccgtattc gcggtgcttc aacgatttct 360

caacagacag ccaaaaatct ctttttatgg gatgggcgta gctgggttcg aaaagggctg 420

gaagccggat taacgctggg gatagaaacg gtctggagca aaaagcgtat cctgacggtt 480

tacctgaata tcgccgaatt tggcgacggt gtgtttggcg tcgaagctgc ggcacaacgt 540

```
tatttccaca aacccgcgag caaacttacc cggtcggaag ctgcattact ggcagctgta      600
ttacctaatc cacttcgttt caaagtctcc tcgccatcgg gctacgtgcg tagccgtcag      660
gcgtggattt tacggcagat gtatcagtta ggcggtgagc cgtttatgca acaacaccag      720
ctggattaa                                                               729
```

## Description

### Technical Field

The present invention relates to a host bacterial strain having a mutation in a mtgA gene used for highly producing a bioplastic, a method for producing the bacterial strain, a bioplastic highly producing bacterial strain having a mutation in a mtgA gene, a method for highly producing a bioplastic comprising culturing the bacterial strain, and a method for producing the bacterial strain.

### Background Art

In recent years, environmentally friendly technology in which consumption of fossil fuel such as petroleum is reduced has attracted attention, and bioplastics have been increasingly made from biomass resources in the world. Microbially produced bioplastics mean bioplastics synthesized from renewable biomass in the cells of microorganisms. The microbially produced bioplastics have been developed as versatile plastic materials, and can be used for environmental or biochemical purposes. Microbial polyesters are a kind of the microbially produced bioplastics, and are generically called polyhydroxyalkanoates (hereinafter, also referred to as PHAs). Many constituent monomers for the microbial polyesters including 3-hydroxybutyrate (hereinafter, also referred to as 3HB) have been reported. Especially, polymers comprising hydroxyalkanoates with about 3 to 14 carbons as constituent monomers are generally used. In particular, development of bioplastics comprising lactic acid monomers such as polylactic acid has advanced toward practical use the most. A pathway for PHA synthesis in microbial cells involves supply of monomers from a metabolic pathway and polymerization of the monomers by a PHA polymerizing enzyme. A monomer-supplying enzyme is involved in the supply of monomers.

To date, as strategies for increasing production of microbially produced bioplastics, introduction of a preferable mutation into the PHA polymerizing enzyme, increase in the expression of a gene encoding the PHA polymerizing enzyme by reinforcement of a promoter, and flux enhancement in intracellular metabolism have been investigated for the purpose of increasing productivity per cell. In addition, conditions for culturing bioplastic-producing bacteria such as pH and a dissolved oxygen level have been investigated. For example, for the purpose of achieving high-yield production of various tailor-made PHAs, recombinant Escherichia coli systems incorporating natural and engineered PHA biosynthesis pathways have been reported (non-patent literatures 1 to 3).

Monofunctional peptidoglycan transglycosylase (hereinafter, also referred to as MtgA) is an enzyme involved in synthesis of peptidoglycan. The peptidoglycan is the main component of a cell wall which forms a layer outside of the intracellular membrane of bacteria, and is known to play an important role in maintaining the cellular morphology. To date, a relationship between MtgA and enhanced production of the microbially produced bioplastics has not been reported.

### Citation List

### Non-patent Literatures

Non-patent Literature 1: Le Meur S. et al., Microb Cell Fact 2014; 13: 131
Non-patent Literature 2: Matsumoto K. et al., J Biotechnol 2011; 156: 214-217
Non-patent Literature 3: Matsumoto K. et al., Biomacromolecules 2013; 14: 1913-1918

### Summary of Invention

### Problems to be solved by the Invention

Further improvement in production of bioplastics by microorganisms is still desired. An objective of the present invention is to develop bacterial strains that produce bioplastics in enhanced yield.

### Solutions to the Problems

In order to further improve production of bioplastics by microorganisms, the present inventors genome-widely introduced a transposon mutation into Escherichia coli cells containing a biosynthesis system for lactate-based polyester poly(lactate-co-3-hydroxybutyrate) [hereinafter, also referred to as P(LA-co-3HB)] and screened a mutant library thus prepared, for the purpose of searching positive factors indirectly contributing to the enhanced production of bioplastics.

As a result, they found that a bacterial strain having a mutation introduced into a genetic sequence encoding monofunctional peptidoglycan glycosyltransferase (MtgA), which is involved in synthesis of peptidoglycan, produced P(LA-co-3HB) in high yield as compared with a control strain (parent strain) that did not contain the mutation. Further interestingly, it was found that the mutant strain swelled its cell morphology and thus increased its cell volume as the bioplastic was synthesized and accumulated in the cell. Thus the present invention was completed.

Specifically, the present invention includes the following aspects:

[1] a host bacterial strain for bioplastic production, comprising a mutation in a gene encoding monofunctional peptidoglycan glycosyltransferase (MtgA), wherein the mutation causes inactivation of MtgA or causes a decrease in activity of MtgA as compared with a control strain that does not comprise the mutation;
[2] a bacterial strain that produces a bioplastic, comprising a mutation in a gene encoding monofunctional peptidoglycan glycosyltransferase (MtgA) and further comprising a group of bioplastic biosynthesis genes, wherein the mutation causes inactivation of MtgA or causes a decrease in activity of MtgA as compared with a control strain that does not comprise the mutation;
[3] the bacterial strain according to [1] or [2], which is Escherichia coli;
[4] the bacterial strain according to any one of [1] to [3], wherein the mutation is deletion of the whole or a part of the nucleotide sequence of the gene encoding MtgA, insertion or substitution of one or more nucleotides in the nucleotide sequence, or a combination thereof;
[5] the bacterial strain according to any one of [2] to [4], wherein the bioplastic is a copolymer comprising lactic acid and 3-hydroxybutyrate, or poly-3-hydroxybutyrate;
[6] the bacterial strain according to any one of [2] to [5], wherein the group of bioplastic biosynthesis genes comprises a gene encoding β-ketothiolase, a gene encoding acetoacetyl-CoA reductase, and a gene encoding PHA synthase;
[7] the bacterial strain according to [6], wherein the group of bioplastic biosynthesis genes further comprises a gene encoding propionyl-CoA transferase;
[8] the bacterial strain according to any one of [2] to [7], which increases cell volume when producing the bioplastic;
[9] a method for producing a bioplastic, the method comprising culturing the bacterial strain according to any one of [2] to [8] under a condition that allows bioplastic production;
[10] a method for producing a host bacterial strain for bioplastic production, the method comprising mutating a gene encoding monofunctional peptidoglycan glycosyltransferase (MtgA), wherein the mutation causes inactivation of MtgA or causes a decrease in activity of MtgA as compared with a control strain that does not comprise the mutation;
[11] a method for producing a bacterial strain that produces a bioplastic, the method comprising mutating a gene encoding monofunctional peptidoglycan glycosyltransferase (MtgA) and introducing a group of bioplastic biosynthesis genes, wherein the mutation causes inactivation of MtgA or causes a decrease in activity of MtgA as compared with a control strain that does not comprise the mutation;
[12] the method according to [10] or [11], wherein the mutation is deletion of the whole or a part of the nucleotide sequence of the gene encoding MtgA, insertion or substitution of one or more nucleotides in the nucleotide sequence, or a combination thereof;
[13] the method according to [11] or [12], wherein the group of bioplastic biosynthesis genes comprises a gene encoding β-ketothiolase, a gene encoding acetoacetyl-CoA reductase, and a gene encoding PHA synthase; and
[14] the method according to [13], wherein the group of bioplastic biosynthesis genes further comprises a gene encoding propionyl-CoA transferase.

Effects of the Invention

According to the present invention, a bacterial strain that highly produces a bioplastic, a method for highly producing a bioplastic which comprises culturing the bacterial strain, and a method for producing the bacterial strain are provided. The bacterial strain of the present invention can stably synthesize bioplastics with high yield from sugar. The bacterial strain of the present invention can increase its cell volume and thus accumulate large amounts of bioplastics. Further, according to the present invention, a bacterial strain usable as a host for highly producing bioplastics is provided, and the bacterial strain is useful as a platform for synthesis of microbially produced bioplastics.

Brief Description of Drawings

[Figure 1] Figure 1 is a schematic diagram showing synthesis of P(LA-co-3HB) by the bacterium that highly produces a bioplastic of the present invention. In the figure, "LDH" means lactate dehydrogenase, "PCT" means propionyl-CoA transferase, "PhaA" means β-ketothiolase, "PhaB" means acetoacetyl-CoA reductase, "PhaC1(ST/QK)" means PHA synthase having a mutation from Ser to Thr at position 325 and a mutation from Gln to Lys at position 481, and "PBPs" means penicillin-binding proteins.

[Figure 2] Figure 2 comprise graphs showing increases in bioplastic production and glucose consumption by the bacterium that highly produces a bioplastic of the present invention. In the figure, bar graphs show the amounts of the produced bioplastic, and line graphs show the amounts of glucose in a medium. (A) shows a result of a control strain having no defect in the mtgA gene. (B) shows a result of the bacterium that highly produces a bioplastic of the present invention.

[Figure 3] Figure 3 comprises graphs showing increases in bioplastic production by the bacterium of the present invention that highly produce a bioplastic. In the figure, "wild-type" means a control strain having no defect in the mtgA gene, and "mutant (ΔmtgA)" means the bacterium that highly produces a bioplastic of the present invention.

Mode for carrying out the Invention

Bacteria used in the present invention are gram-negative bacteria. Examples of the gram-negative bacteria include bacteria belonging to the genus Pseudomonas, the genus Acinetobacter, the genus Actinobacillus, the genus Agrobacterium, the genus Alcallgenes, the genus Bordetella, the genus Brucella, the genus Escherichia, the genus Klebsiella, the genus Proteus, the genus Rhizobium, the genus Rhodobacter, the genus Salmonella, and the genus Vibrio. Preferably, Escherichia coli is used.

The bacterium of the present invention comprises a mutation in a gene encoding monofunctional peptidoglycan glycosyltransferase (MtgA) (hereinafter, also referred to as "the mtgA gene"), wherein the mutation causes inactivation of MtgA or causes a decrease in activity of MtgA as compared with a control strain that does not comprise the mutation. As used herein, the mutation is also referred to as "the defect in the mtgA gene". The "control strain that does not comprise the mutation" means a strain comprising no mutation in the mtgA gene (in other words, having no defect in the mtgA gene), for example, a wild-type strain or a parent strain before the mutation is introduced. The "inactivation" includes loss, suppression and decrease of the activity. In other words, the "inactivation of MtgA" includes loss, suppression and decrease of the MtgA activity, loss, suppression and decrease of the expression of MtgA, and loss, suppression and decrease of the function of the gene encoding MtgA. The MtgA is an enzyme involved in synthesis of peptidoglycan. In the present invention, the mutation in the gene encoding MtgA may be any mutation that causes inactivation of MtgA or a decrease in the MtgA activity. The mutation can be introduced by a method known in the art. Examples of the mutation include deletion of the whole or a part of the nucleotide sequence of the gene encoding MtgA, insertion or substitution of one or more nucleotides in the nucleotide sequence, or a combination thereof; insertion of at least one stop codon in the gene encoding MtgA; insertion or deletion of one or more nucleotides for introduction of a frameshift mutation; and introduction of one or more point mutations in the gene encoding MtgA.

The mutation is introduced into the gene encoding MtgA of the above-mentioned bacterium, and the bacterium thus obtained can be used as a host bacterium for bioplastic production (hereinafter, also referred to as "the host bacterial strain of the present invention"). Into the host bacterium, a group of desired bioplastic biosynthesis genes are introduced, and thereby a bioplastic-producing bacterium is obtained. Alternatively, in the present invention, the mutation may be introduced into the gene encoding MtgA of a bacterium having a group of bioplastic biosynthesis genes. The bioplastic-producing bacterium thus obtained is also referred to as "the bacterial strain of the present invention".

Bioplastics mean plastics produced from biomass resources such as starch and sugar. As used herein, the bioplastics mean microbially produced bioplastics which are produced by microorganisms in their bodies. Examples of the microbially produced bioplastics include polyesters such as polylactic acid, polybutylene succinate, and polyhydroxyalkanoates.

As used herein, the "group of bioplastic biosynthesis genes" means two or more enzymes necessary for synthesis of bioplastics within bacteria, for example two or more genes encoding a monomer-supplying enzyme and a PHA-polymerizing enzyme. Examples of the monomer-supplying enzyme include β-ketothiolase (PhaA), acetoacetyl-CoA reductase (PhaB), enoyl-CoA hydratase (PhaJ), and propionyl-CoA transferase (PCT). Examples of the PHA-polymerizing enzyme include PHA synthase (PhaC1), lactate-polymerizing enzyme (LPE), class 3 PHA synthase subunit (PhaE), and class 4 PHA synthase (PhaR). These enzymes may be native enzymes or mutant-type enzymes. Examples of the mutant-type enzymes include mutants of PhaC1, for example, a PHA synthase having a mutation from Ser to Thr at position 325 and a mutation from Gln to Lys at position 481 [phaC1(ST/QK)], and a PHA synthase having a mutation from Phe to Ser at position 392 of the phaC1(ST/QK) [phaC1(ST/FS/QK)]. The phaC1(ST/QK) is also used as a lactate-polymerizing enzyme. Examples of genes encoding the above-mentioned enzymes include β-ketothiolase genes, for example, derived from the genus Ralstonia, such as Ralstonia eutropha; acetoacetyl-CoA reductase genes, for example, derived from the genus Zoogloea, such as Zoogloea ramigera; enoyl-CoA hydratase genes, for example, derived from the genus Pseudomonas, such as Pseudomonas aeruginosa; propionyl-CoA transferase genes, for example, derived from the genus Megasphaera, such as Megasphaera elsdenii, the genus Staphylococcus, such as Staphylococcus aureus, and the genus Clostridium, such as Clostridium propionicum; and PHA synthase genes, for example, derived from the genus Pseudomonas, such as Pseudomonas sp. 61-3, and Pseudomonas aeruginosa, the genus Alcanivorax, such as Alcanivorax borkumensis, and the genus Synechocystis, such as Synechocystis sp. PCC6803. The genes of the bioplastic biosynthesis gene group may be endogenous genes of the bioplastic-producing bacteria or foreign genes. All of the genes of the bioplastic biosynthesis gene group may be endogenous genes of the bioplastic-producing bacteria, or all of the genes of the bioplastic biosynthesis gene group may be foreign genes, or some of the genes of the bioplastic biosynthesis gene group may be endogenous genes and the remainder may be foreign genes.

When the bioplastic biosynthesis gene is a foreign gene, the gene may be introduced into the host bacterial cell by conventional genetic recombination technology using a vector or the like. Examples of the vector for introduction of the gene include vectors capable of autonomous replication in hosts, for example, plasmid DNA, phage DNA, and the like. The gene to be introduced into the host bacterial cell may be connected downstream of a suitable promoter. The promoter may be any promoter that regulates gene transcription within the host bacterium, and can be selected depending on the host cell used. In the vector, an enhancer sequence, a terminator sequence, a selective marker gene and the like can be connected, if necessary. The vector may be introduced into the host bacterium by a conventional method known to a person skilled in the art. Examples of the introduction method include a conjugational transfer method, a calcium phosphate method, an electroporation method, a spheroplast method, and a lithium acetate method.

As a result of introduction of the bioplastic biosynthesis gene group into the host bacterium, the bioplastic biosynthesis pathway is conferred to the bacterium, and thus the bacterium becomes capable of producing bioplastics.

The group of bioplastic biosynthesis genes can be appropriately selected depending on the kind of a bioplastic desired to be produced. For example, when the desired bioplastic is P(LA-co-3HB), the bioplastic biosynthesis gene group comprises, for example, a group of genes encoding PCT, PhaA, PhaB, and PhaC1(ST/QK). For example, when the desired bioplastic is P(3HB), the bioplastic biosynthesis gene group comprises, for example, a group of genes encoding PhaA, PhaB, and PhaC1(ST/QK).

Examples of bioplastics produced by the bacterial strain of the present invention include, but not limited to, polyesters, for example, polylactic acid, P(LA-co-3HB), poly-3-hydroxybutyrate (hereinafter, also referred to as P3HB), copolymers of 3HB and other hydroxyalkanoates, for example, copolymers of 3HB and hydroxyacetic acid [P(GL-co-3HB)], poly(2-hydroxybutyrate) [P(2HB)], poly(3-hydroxybutyrate-co-3-hydroxyvalerate) [P(3HB-co-3HV)], and poly(3-hydroxybutyrate-co-3-hydroxyhexanoate [P(3HB-co-3HHx)].

The bioplastics can be produced by culturing the bacterial strain obtained as described above, comprising the group of bioplastic biosynthesis genes and comprising, in the gene encoding MtgA, a mutation that causes inactivation of MtgA or causes a decrease in the MtgA activity as compared with a control strain having no mutation, in a medium containing a carbon source. The bioplastics are produced and accumulated within the bacterial cell.

Examples of the carbon source to be contained in the medium include carbohydrates, such as glucose, galactose, fructose, sucrose, and maltose. In addition the medium may contain a nitrogen source, for example ammonium chloride, and an inorganic substance, for example potassium dihydrogen phosphate, sodium chloride, magnesium sulfate, sodium dihydrogen phosphate, or calcium chloride. For example, as the medium for culturing the bacterial strain of the present invention, an LB medium containing glucose and calcium pantothenate can be used. In addition, an antibiotic such as kanamycin, ampicillin, or chloramphenicol may be added to the medium as appropriate.

Conditions for culturing the bacterial strain of the present invention in the medium can be appropriately determined depending on the kind of the bacterium used. For example, the bacterial strain is cultured under an aerobic condition such as shaking, at 25°C to 37°C for 24 hours or more. Depending on the kind of the bacterium used, the bacterial strain is preferably cultured in an LB medium containing sugar (glucose final concentration: 2%) under an aerobic condition at 30°C for 48 hours.

The bioplastics thus produced and accumulated within the bacterial cells can be collected by a method known to a person skilled in the art. For example, the bacterial cells can be collected by centrifugation of the culture fluid, dried, and extracted with chloroform or the like, and then, the extract can be purified.

When the bacterial strain of the present invention is cultured under suitable conditions as described above, the bacterial strain produces an increased amount of a bioplastic as compared with the control strain having no mutation in the gene encoding MtgA. Further, it was observed that the bacterial strain of the present invention enlarged the cell size when producing the bioplastic. Such enlargement of the cell size more remarkably increased the cell width (short axis) (y) than the cell length (long axis) (x). Thus, the cell having enlarged size has a swollen form like a balloon (hereinafter, also referred to as a fat cell). In contrast, when the bacterial strain of the present invention that did not contain the group of bioplastic biosynthesis genes (which was obtained by introducing a plasmid that did not contain the group of bioplastic biosynthesis genes into the host bacterial strain of the present invention; hereinafter, also referred to as "the bioplastic-nonproductive conditions") was cultured under the above-mentioned suitable culture conditions, such enlargement of the cell size was not observed and a form equivalent to the control strain was observed. Thus, it was found that the effect on the cell size by a mutation in the gene encoding MtgA was linked with the biosynthesis and accumulation of bioplastics. Such enlargement of the cell size is probably caused by outward pressure from the bioplastic synthesized in the cell. Such a mutation in a single gene as induces both increase in the bioplastic production and enlargement of the cell size has never been reported before as far as the present inventors know, and it is found for the first time.

Examples

Hereinafter, the present invention is explained in detail by reference to the following Examples, to which the present invention is not limited.

Example 1

[Materials and Methods]

1. Plasmids, strains, and growth conditions

Escherichia coli (E. coli) strains used in Examples are shown in Table 1. Expression vector pTV118NpctphaC1$_{Ps}$(ST/QK)AB harboring genes encoding propionyl-CoA transferase from Megasphaera elsdenii (pct), engineered PHA synthase having lactic acid-polymerizing activity [phaC1$_{Ps}$(ST/QK)] from Pseudomonas sp. 61-3, and 3HB-CoA supplying enzymes β-ketothiolase and acetoacetyl-CoA reductase (phaA and phaB, respectively) from Ralstonia eutropha was prepared, and used for production of P(LA-co-3HB). Methods for preparing the vector and the like are as described before (see References 1 to 4). Modeled synthetic pathways for P(LA-co-3HB) are shown in Figure 1. As a helper plasmid for conjugation, pTS52 was used (see Reference 5).

For bioplastic production, recombinant E. coli harboring the pTV118NpctphaC11$_{Ps}$(ST/QK)AB was cultured on 1.7 mL of an LB medium containing 20 g/l glucose and 10 mM calcium pantothenate at 30°C for 48 hours with shaking at 180 rpm. To the medium, 100 μg/ml ampicillin (Amp), 25 μg/ml kanamycin (Km) and 25 μg/ml chloramphenicol (Cm) were added as necessary. pCA24N-mtgA was obtained from ASKA clone. As the bioplastic-nonproductive conditions, E. coli was transformed with plasmid pTV118N (manufactured by TAKARA BIO INC.) that did not contain a group of bioplastic biosynthesis genes, and cultured under the same culture conditions as described above.

[Table 1]

| Table 1. E. coli strains used in the present invention | | | |
|---|---|---|---|
| Strains | Genotype | Purpose | References |
| S17-1 | MG1655 RP4-2-tc::[ΔMu1::aac(3)IV-ΔaphA-Δnic35-ΔMu2::zeo]ΔdapA:: (erm-pir) ΔrecA | Transposon mutagenesis of JM109 | 6, 7 |
| JM109 | endA1 glnV44 thi-1 relA1 gyrA96 recA1 mcrB+ Δ(lac-proAB) e14- [F' traD36 proAB+ lacIq lacZΔM15]hsdR17(rK-mK+) | Host strain of transposon mutagenesis | 7 |
| JM109 C21 | JM109 ΔmtgA::Tn5 | Selected mutant of JM109 as bioplastic highly-produced strain | The present invention |
| BW25113 | Δ(araD-araB) 567 Δlac24787(::rrnB-3) lacIp-4000(laclq) λ-rph-1 Δ(rhaD-rhaB)568 hsdR514 | Parent strain of Keio collection mutants | 8 |
| JW3175 | ΔmtgA::FRT-kan-FRT | Mutant in Keio collection | 8 |

2. Construction of mutant library of E. coli JM109 and screening for positive mutants

A library of mutants of E. coli strain JM109 producing P(LA-co-3HB) was prepared by using transposon mini-Tn5. The pTV118NpctphaC11$_{Ps}$(ST/QK)AB (Amp-resistant) and the pST52 (Cm-resistant) were introduced into E. coli JM109 to obtain a recombinant. Using E. coli S17-λ-A-pir into which pUTmini-Tn5 Km (Amp-resistant; Biomedical, Seville, Spain; see Reference 9) had been introduced, mini-Tn5 transposon (Km-resistant) was conjugationally transferred to the recombinant E. coli JM109 harboring the pTV118NpctphaC11$_{Ps}$(ST/QK)AB (Amp-resistant) and the pST52 (Cm-resistant). The S17-1 and JM109 cells were conjugated on an LB agar plate at 30°C for 16 hours. The cells were suspended in 10 mM $MgSO_4$, and cultured on LB plates containing Cm, Km and Amp. The presence of Cm eliminated the S17-1 cells. Km was used to select transposon-inserted JM109 cells. Amp was used to maintain the pTV118NpctphaC1$_{Ps}$(ST/QK)AB. The plates were incubated at 30°C for 16 hours. The transposon-inserted JM109 cells harboring the pTV118NpctphaC1$_{Ps}$(ST/QK)AB were screened on LB plates containing 2% glucose, Amp, Km and 0.5

μg/ml Nile red (manufactured by Sigma-Aldrich). Colonies emitting strong fluorescence were chosen as candidates under a transilluminator, and subjected to HPLC analysis to measure the amount of bioplastic production as described before (see Reference 10). In brief, the cells were directly treated with concentrated sulfuric acid at 120°C to convert polyester into unsaturated crotonic acid, which was measured at 210 nm using a UV detector (see References 11 and 12). The concentration of glucose in a culture supernatant was measured by HPLC equipped with a refractive index detector as described before (see Reference 13).

3. Identification of transposon insertion site

A transposon insertion site was identified using an inverse PCR method. The chromosomal DNA of E. coli was digested with PstI, self-ligated, and amplified by PCR using a pair of primers of 5'-AAGGTGATCCGGTGGATGAC-3' (SEQ ID NO:1) and 5'-CAATCGGCTGCTCTGATGCCGC-3' (SEQ ID NO:2) which annealed to the Km-resistant gene in the transposon (see References 14 and 15). Amplified fragments were sequenced to identify the transposon insertion site in the E. coli chromosome.

4. Measurement of cell density using flow cytometry

Volumetric cell density (cells/l) was measured by flow cytometry using an SH800 cell sorter (manufactured by SONY). The cells were grown under the above-described conditions for 48 hours, collected ($OD_{600}$ between 20 and 25), diluted 10 times with water, and then subjected to analysis. A flow rate was 11 μl/min (pressure 2). All FSC (forward scatter) and SSC (side scatter) images were recorded using SH800 software (manufactured by SONY).

5. Determination of cell size

The cells were grown under the bioplastic-productive conditions for 48 hours and then harvested. Images of the cells were captured using microscope BZA-X700 (manufactured by Keyence). On the digital images, the cell length (long axis) (x) and the cell width (short axis) (y) of 150 to 200 cells under each condition were measured using ImageJ software (http://rsb.info.nih.gov/ij/index.html). Based on the "lemon-shaped" morphology of bioplastic-accumulating cells, the cell volume (V) closely resembled the volume of an oval sphere. Thus the cell volume was calculated using the following formula (1).
[Numerical formula 1]

$$V = \frac{4}{3}\pi \cdot \frac{x}{2} \cdot \left(\frac{y}{2}\right)^2 \qquad (1)$$

[Results]

1. Transposon mutagenesis and screening of highly bioplastic-producing mutant

As described above, the library of mutants of E. coli strain JM109 producing P(LA-co-3HB) was prepared by using transposon mini-Tn5. High throughput screening for bioplastic-accumulating cells was performed by means of plate assay using Nile red-containing agar plates. The dye staining enabled easy screening for candidates that produced bioplastics in larger amounts than bioplastic production amounts by the original recombinant (the parent control). Since JM1099 was efficiently stained with Nile red, the strain was used as a host. Of about 10,000 colonies, 100 colonies were chosen as candidates at the first stage. The bioplastic production amounts of the candidates were determined using HPLC. Eventually, one mutant C21 which showed enhanced bioplastic accumulation was isolated. The mutant C21 showed an increased amount of bioplastic production (5.1 g/l) as compared with the bioplastic production amount (2.9 g/l) of the parent recombinant.

2. Contribution of defect in mtgA gene to enhanced bioplastic production

The chromosomal DNA of C21 was subjected to nucleotide sequencing analysis, and as a result, it was found that the transposon was inserted between nucleotide 98 and nucleotide 99 in the nucleotide sequence of the mtgA gene set forth by SEQ ID NO:3. In order to confirm that the mutation in the mtgA gene related to enhanced bioplastic production, P(LA-co-3HB)-producing E. coli strain BW25113, which was widely used for bioplastic production, was used to perform the following experiments (see References 10 and 16).

BW25113, and JW3175 which was an mtgA-defective strain of BW25113 (wherein the mtgA gene region was replaced by a kanamycin resistance gene) were obtained from Keio collection (see References 8 and 17). The pTV118NpctphaC1$_{Ps}$(ST/QK)AB was introduced into JW3175 and BW25113 by a conventional method. The recombinants of JW3175 and BW25113 thus obtained (hereinafter, also referred to as "rJW" and "the parent recombinant" respectively) were cultured on an LB medium containing 20 g/l glucose at 30°C for 48 hours with shaking at 180 rpm to induce accumulation of bioplastics (Table 2). Similarly to C21, rJW produced P(LA-co-3HB) in an increased amount (7.0 g/l) as compared with the bioplastic production amount (5.2 g/l) of the parent recombinant.

In order to confirm the contribution of the mtgA defect to the enhanced bioplastic production, a complementary experiment was performed wherein rJW was complemented with the expression of an exogenous mtgA gene (see Reference 18). As a result of the complement, the phenotype of the parent recombinant was recovered (Table 2). Thus, it was found that the defect in the mtgA gene caused an increase of P(LA-co-3HB) production in E. coli. In contrast, the defect in the mtgA gene hardly affected the LA/3HB ratio in the copolymer.

[Table 2]

| Table 2. P(LA-co-3HB) production in mtgA-defective and complemented strains | | | | | | |
|---|---|---|---|---|---|---|
| Genoty pe | Plasmid | Cell dry weight (g/l) | True cell weight (g/l) | Bioplastic production (g/l) | | |
| | | | | Total | LA | 3HB |
| Wild type | pTV118Npct phaC1$_{Ps}$(ST/QK)AB | 9.2±0.2 | 4.1±0.3 | 5.1±0. 4 | 0.8±0. 1 | 4.3±0. 3 |
| ΔmtgA | pTV118Npct phaC1$_{Ps}$(ST/QK)AB | 11.6±1.0 | 4.3±0.9 | 7.3±0. 3 | 1.0±0. 2 | 6.3±0. 1 |
| ΔmtgA[a] | pTV118Npct phaC1$_{Ps}$(ST/QK)AB + pCA24N-mtgA | 8.0±0.7 | 3.2±0.3 | 4.9±0. 3 | 0.9±0. 1 | 3.9±0. 3 |

In Table, the wild type means E. coli BW25113 harboring the pTV118NpctphaC1$_{Ps}$(ST/QK)AB, and ΔmtgA means JW3175 he pTV118NpctphaC1$_{Ps}$(ST/QK)AB. The data present the average ± standard deviation of three independent experiments. The pCA24N-mtgA bears the mtgA gene expressed by a lac promoter. The a means that 100 μM IPTG was added. The true cell weight is calculated by subtracting the bioplastic weight from the dry weight of the whole cell.

Time profiles of bioplastic accumulation and glucose consumption are shown in Figure 2. In Figure 2, "Wild type/pTV118NpctphaC1$_{Ps}$(ST/QK)AB" means the parent recombinant (Figure 2(A)), and "Mutant (ΔmtgA)/pTV118NpctphaC1$_{Ps}$(ST/QK)AB" means rJW (Figure 2(B)). At the initial stage, rJW produced a slightly smaller amount of the bioplastic than the parent recombinant. However, 24 hours after, rJW attained a larger production amount than the parent recombinant. Particularly, rJW consumed glucose more rapidly than the parent recombinant. The amount of glucose consumption by rJW was 20 g/l after 48 hours of culturing (Figure 2), and the amount of bioplastic production by rJW was 7.3 g/l (Figure 2). In contrast, the amount of glucose consumption by the parent recombinant (wild type) was about 16.5 g/l after 48 hours of culturing (Figure 2), and the amount of bioplastic production by the parent recombinant (wild type) was 5.1 g/l (Figure 2). The yield of the bioplastic from glucose in rJW (0.37 g/g) was higher than the bioplastic yield (0.31 g/g) in the parent recombinant. This shows that rJW has higher use efficiency of glucose than the parent recombinant. Thus, the defect in mtgA increased both glucose consumption and conversion efficiency into bioplastics. A bioplastic yield from glucose was calculated using the following equation.

Bioplastic yield from glucose (g/g) = Bioplastic production (g/l) / Glucose consumption (g/l)

3. Cell size enlargement caused by bioplastic accumulation in mtgA-defective strain

The pTV118NpctphaC11$_{Ps}$(ST/QK)AB was introduced into E. coli BW25113 (wild type) and JW3175 (ΔmtgA), and the recombinants (bioplastic-productive conditions) thus obtained were grown on an LB medium at 30°C for 48 hours. As controls, the pTV118N was introduced into E. coli BW25113 (wild type) and JW3175 (ΔmtgA), and the recombinants (bioplastic-nonproductive conditions) thus obtained were cultured in the same way. Cell density was measured using flow cytometry. The size of each cell was determined using microscopic images of 150 to 200 cells. The transposon-inserted strain JM109 C21 and the complemented strain of rJW were also cultured in the same way, and the cell size was determined.

When the cell morphology of the mtgA-defective strain having bioplastic accumulation was observed, under the bioplastic-nonproductive conditions, JW3175 had similar cell size to that of the parent strain BW25113 (Table 3). Thus, it was found that the mtgA defect did not alone affect the cell morphology. In contrast, under the bioplastic-productive conditions, the rJW cell remarkably increased the cell size (1.4 times) as compared with the parent recombinant (Table 3). The mtgA defect caused an increase only in the cell width (short axis) (y), and did not cause an increase in the cell length (long axis) (x). Thus, the cell had a swollen shape rather than a long shape. Furthermore, the complemented strain of rJW had similar cell size to that of the parent recombinant. This supports that the mtgA defect contributed to the cell enlargement. The transposon-inserted strain JM109 C21 had similar cell morphology to that of rJW.

[Table 3]

| Table 3. Correlation between bioplastic production and cell volume in recombinant E. coli having mtgA defect | | | | | | | | |
|---|---|---|---|---|---|---|---|---|
| Genot ype | Plasmid | Cell density ($l^{-1}$) | Single cell dry weight (g) | Cellular biopl astic conte nt (wt%) | Bioplast ic producti on per single cell (g) | Cell size | | |
| | | | | | | Cell length (μm) | Cell widt h (μm) | Volu me ($\mu m^3$) |
| Wild type | pTV118N | $0.73\times10^{12}$ | $2\times10^{-12}$ | N.D. | N.D. | 2.26 ± 0.56 | 1.10 ± 0.12 | 1.43 |
| Wild type | pTV118N pctC1 (S TQK) AB | $1.2\times10^{12}$ | $8\times10^{-12}$ | 56.8 ± 1.8 | $4.5\times10^{-12}$ | 3.36 ± 0.82 | 1.15 ± 0.16 | 2.56 |
| ΔmtgA | pTV118N | $0.75\times10^{12}$ | $2\times10^{-12}$ | N.D. | N.D. | 2.49 ± 0.62 | 1.14 ± 0.23 | 1.69 |
| ΔmtgA | pTV118N pctC1 (S TQK) AB | $0.96\times10^{12}$ | $12\times10^{-12}$ | 60.9 ± 3.5 | $7.3\times10^{-12}$ | 3.48 ± 0.84 | 1.42 ± 0.25 | 3.67 |

The data present the average ± standard deviation of three independent experiments. The cellular bioplastic content is defined as the percentage of the bioplastic weight in the total cell dry weight. N.D. means that it was not detectable.

4. Quantitative analysis of bioplastic production in fat cell

The bioplastic production (P) was determined using the following equation.

$$P\ (g/l) = \text{Cell density (cells/l)} \times \text{Single cell weight (g/cell)} \times \text{Cellular bioplastic content (wt\%)}$$

In order to gain an advantage of the cell enlargement, an effect on cell growth is an important factor. The growth of rJW was slightly slower than the control at the initial stage (Figure 2). However, after 48 hours of culturing, a decrease in the cell density (cell number per volume) of the bioplastic-accumulating rJW compared to the parent recombinant was as small as 20% (Table 3). This suggests that there was no severe effect of the mtgA defect on cell growth. In addition, the mtgA-defective strain appeared to be robust, and the cell lysis was not observed.

The bioplastic production per cell is also important. Based on the cell dry weight and cellular bioplastic content, the bioplastic production in a single cell of rJW was estimated at $7.3 \times 10^{-12}$ g/cell. This was 1.6 times higher than that in the parent recombinant ($4.5 \times 10^{-12}$ g/cell). This result clearly shows that the rJW fat cell has high capacity to accumulate bioplastics. The increase in cell size necessarily is accompanied by an increase in the amount (area) of a cell membrane. In fact, the true cell weight (the weight obtained by subtracting the weight of bioplastics from the total cell dry weight) of rJW was heavier than that of the parent recombinant, when the cells accumulated bioplastics (Table 3). However, the benefit from the increased capacity to accumulate PHA outweighed additional consumption of carbon sources for cell formation, and overall, the bioplastic production (g/l) and the bioplastic yield (g/g) to glucose consumption (g/l) in rJW were increased. Conversely, this fact shows that the size of intracellular space was a limiting factor for bioplastic accumulation in E. coli, and the mtgA defect loosened the limitation.

Example 2

E. coli BW25113 and JW3175 were transformed with plasmid pGEM-C1$_{Ps}$(ST/QK)AB. The plasmid contains genes encoding PhaA, PhaB and PhaC1$_{Ps}$(ST/QK) of the bioplastic biosynthesis genes, and gives the ability to synthesize P(3HB) to cells into which the plasmid has been introduced. The preparation and transformation of the plasmid were performed as described before (see References 1 to 4) and following conventional methods. The recombinant E. coli thus obtained was cultured and the bioplastic production amount was measured in the same way as Example 1. Results are shown in Figure 3, together with the results of P(LA-co-3HB) production obtained by Example 1. In Figure 3, "Wild type" means a recombinant of BW25113, and "Mutant (ΔmtgA)" means the recombinant of JW3175.

In Example 1, the P(LA-co-3HA) production in the recombinant E. coli was increased by destruction of the mtgA gene which induced the formation of fat cells (Figure 2). The same event was observed for the P(3HB) production in the mtgA-defective strain in this Example. This shows that the advantageous effect of mtgA defect is not limited to the production of P(LA-co-3HB), and is applicable to a wide variety of intracellularly accumulated compounds.

Industrial Applicability

According to the present invention, a bacterial strain that highly produces a bioplastic, a method for highly producing a bioplastic which comprises culturing the bacterial strain, and a method for producing the bacterial strain are provided. The bacterial strain of the present invention can stably synthesize bioplastics with high yield from sugar. The bacterial strain of the present invention can increase its cell volume and thus accumulate large amounts of bioplastics. Further, according to the present invention, a bacterial strain usable as a host for highly producing bioplastics is provided, and the bacterial strain is useful as a platform for synthesis of microbially produced bioplastics. The bacterial strain of the present invention produces large amounts of bioplastics and efficiently uses glucose to produce bioplastics, as compared with the wild type strain. Larger amounts of bioplastics can be produced from smaller amounts of substrates (biomass resources such as starch and sugar) by using the bacterial strain of the present invention.

References (These references are incorporated herein by reference.)

1. Elsden SR, Gilchrist FM, Lewis D, Volcani BE. Properties of a fatty acid forming organism isolated from the rumen of sheep. J Bacteriol 1956; 72: 681-689.
2. Matsusaki H, Manji S, Taguchi K, Kato M, Fukui T, Doi Y. Cloning and molecular analysis of the Poly(3-hydroxybutyrate) and Poly(3-hydroxybutyrate-co-3-hydroxyalkanoate) biosynthesis genes in Pseudomonas sp. strain 61-3. J Bacteriol 1998; 180: 6459-6467.
3. Yamada M, Matsumoto K, Shimizu K, Uramoto S, Nakai T, Shozui F, et al. Adjustable mutations in lactate (LA)-polymerizing enzyme for the microbial production of LA-based polyesters with tailor-made monomer composition. Biomacromolecules 2010; 11: 815-819.
4. Taguchi S, Yamada M, Matsumoto K, Tajima K, Satoh Y, Munekata M, et al. A microbial factory for lactate-based polyesters using a lactate-polymerizing enzyme. Proc Natl Acad Sci U S A 2008; 105: 17323-17327.
5. Som T, Tomizawa J. Origin of replication of Escherichia coli plasmid RSF 1030. Mol Gen Genet 1982; 187: 375-383.
6. Kadoya R, Baek JH, Sarker A, Chattoraj DK. Participation of chromosome segregation protein ParAI of Vibrio cholerae in chromosome replication. J Bacteriol 2011; 193: 1504-1514
7. Yanisch-Perron C, Vieira J, Messing J. Improved M13 phage cloning vectors and host strains: nucleotide sequences of the M13mp18 and pUC19 vectors. Gene 1985; 33: 103-119
8. Baba T, Ara T, Hasegawa M, Takai Y, Okumura Y, Baba M, et al. Construction of Escherichia coli K-12 in-frame, single-gene knockout mutants: the Keio collection. Mol Syst Biol 2006; 2: 2006 0008
9. de Lorenzo V, Herrero M, Jakubzik U, Timmis KN. Mini-Tn5 transposon derivatives for insertion mutagenesis, promoter probing, and chromosomal insertion of cloned DNA in gram-negative eubacteria. J Bacteriol 1990; 172: 6568-6572.
10. Nduko JM, Matsumoto K, Ooi T, Taguchi S. Enhanced production of poly(lactate-co-3-hydroxybutyrate) from xylose in engineered Escherichia coli overexpressing a galactitol transporter. Appl Microbiol Biotechnol 2014; 98: 2453-2460.
11. Kichise T, Taguchi S, Doi Y. Enhanced accumulation and changed monomer composition in polyhydroxyalkanoate (PHA) copolyester by in vitro evolution of Aeromonas caviae PHA synthase. Appl Environ Microbiol 2002; 68: 2411-2419.

12. Spiekermann P, Rehm BH, Kalscheuer R, Baumeister D, Steinbuechel A. A sensitive, viable-colony staining method using Nile red for direct screening of bacteria that accumulate polyhydroxyalkanoic acids and other lipid storage compounds. Arch Microbiol 1999; 171: 73-80.

13. Matsumoto K, Okei T, Honma I, Ooi T, Aoki H, Taguchi S. Efficient (R)-3-hydroxybutyrate production using acetyl CoA-regenerating pathway catalyzed by coenzyme A transferase. Appl Microbiol Biotechnol 2013; 97: 205-210.

14. Sambrook J RD. Molecular Cloning: A Laboratory Manual, 3rd edn. Cold Spring Harbor, NY. Cold Spring Harbor Laboratory 2001.

15. Washio K, Lim SP, Roongsawang N, Morikawa M. Identification and characterization of the genes responsible for the production of the cyclic lipopeptide arthrofactin by Pseudomonas sp. MIS38. Biosci Biotechnol Biochem 2010; 74: 992-999.

16. Nduko JM, Matsumoto K, Ooi T, Taguchi S. Effectiveness of xylose utilization for high yield production of lactate-enriched P(lactate-co-3-hydroxybutyrate) using a lactate-overproducing strain of Escherichia coli and an evolved lactate-polymerizing enzyme. Metab Eng 2013; 15: 159-166.

17. Datsenko KA, Wanner BL. One-step inactivation of chromosomal genes in Escherichia coli K-12 using PCR products. Proc Natl Acad Sci U S A 2000; 97: 6640-6645.

18. Kitagawa M, Ara T, Arifuzzaman M, Ioka-Nakamichi T, Inamoto E, Toyonaga H, et al. Complete set of ORF clones of Escherichia coli ASKA library (a complete set of E. coli K-12 ORF archive): unique resources for biological research. DNA Res 2005; 12: 291-299.

## Claims

1. A host bacterial strain for bioplastic production, comprising a mutation in a gene encoding monofunctional peptidoglycan glycosyltransferase (MtgA), wherein the mutation causes inactivation of MtgA or causes a decrease in activity of MtgA as compared with a control strain that does not comprise the mutation.

2. A bacterial strain that produces a bioplastic, comprising a mutation in a gene encoding monofunctional peptidoglycan glycosyltransferase (MtgA) and further comprising a group of bioplastic biosynthesis genes, wherein the mutation causes inactivation of MtgA or causes a decrease in activity of MtgA as compared with a control strain that does not comprise the mutation.

3. The bacterial strain according to claim 1 or 2, which is Escherichia coli.

4. The bacterial strain according to any one of claims 1 to 3, wherein the mutation is deletion of the whole or a part of the nucleotide sequence of the gene encoding MtgA, insertion or substitution of one or more nucleotides in the nucleotide sequence, or a combination thereof.

5. The bacterial strain according to any one of claims 2 to 4, wherein the bioplastic is a copolymer comprising lactic acid and 3-hydroxybutyrate, or poly-3-hydroxybutyrate.

6. The bacterial strain according to any one of claims 2 to 5, wherein the group of bioplastic biosynthesis genes comprises a gene encoding β-ketothiolase, a gene encoding acetoacetyl-CoA reductase, and a gene encoding PHA synthase.

7. The bacterial strain according to claim 6, wherein the group of bioplastic biosynthesis genes further comprises a gene encoding propionyl-CoA transferase.

8. The bacterial strain according to any one of claims 2 to 7, which increases cell volume when producing the bioplastic.

9. A method for producing a bioplastic, the method comprising culturing the bacterial strain according to any one of claims 2 to 8 under a condition that allows bioplastic production.

10. A method for producing a host bacterial strain for bioplastic production, the method comprising mutating a gene encoding monofunctional peptidoglycan glycosyltransferase (MtgA), wherein the mutation causes inactivation of MtgA or causes a decrease in activity of MtgA as compared with a control strain that does not comprise the mutation.

11. A method for producing a bacterial strain that produces a bioplastic, the method comprising mutating a gene encoding monofunctional peptidoglycan glycosyltransferase (MtgA) and introducing a group of bioplastic biosynthesis genes, wherein the mutation causes inactivation of MtgA or causes a decrease in activity of MtgA as compared with a control strain that does not comprise the mutation.

12. The method according to claim 10 or 11, wherein the mutation is deletion of the whole or a part of the nucleotide sequence of the gene encoding MtgA, insertion or substitution of one or more nucleotides in the nucleotide sequence, or a combination thereof.

[Fig. 1]

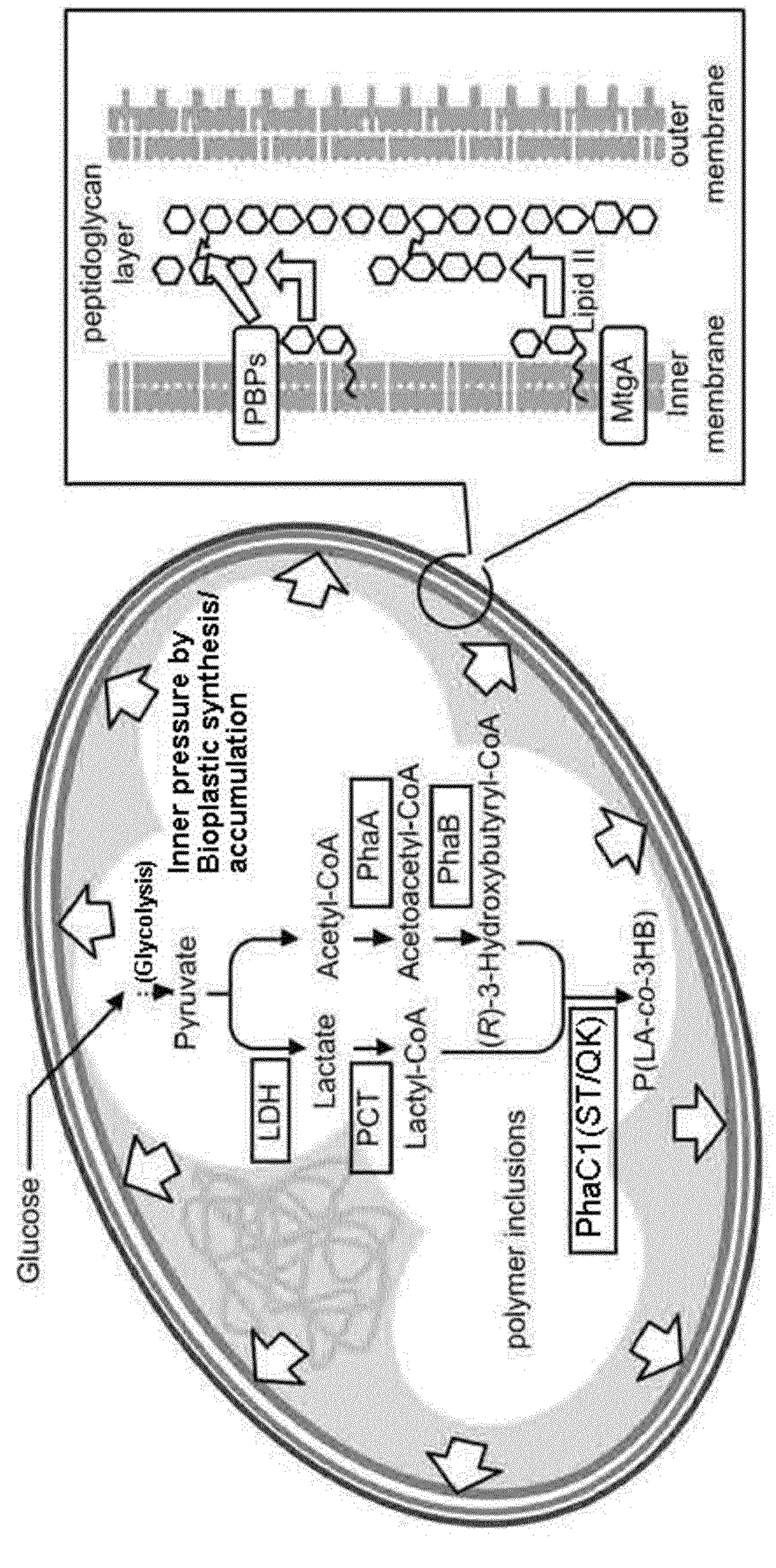
peptidoglycan layer
outer membrane
PBPs
Lipid II
MtgA
Inner membrane
Inner pressure by Bioplastic synthesis/ accumulation
Glucose
(Glycolysis)
Pyruvate
LDH
Lactate
Acetyl-CoA
PhaA
PCT
Lactyl-CoA
Acetoacetyl-CoA
PhaB
(R)-3-Hydroxybutyryl-CoA
PhaC1(ST/QK)
P(LA-co-3HB)
polymer inclusions

[Fug. 2]

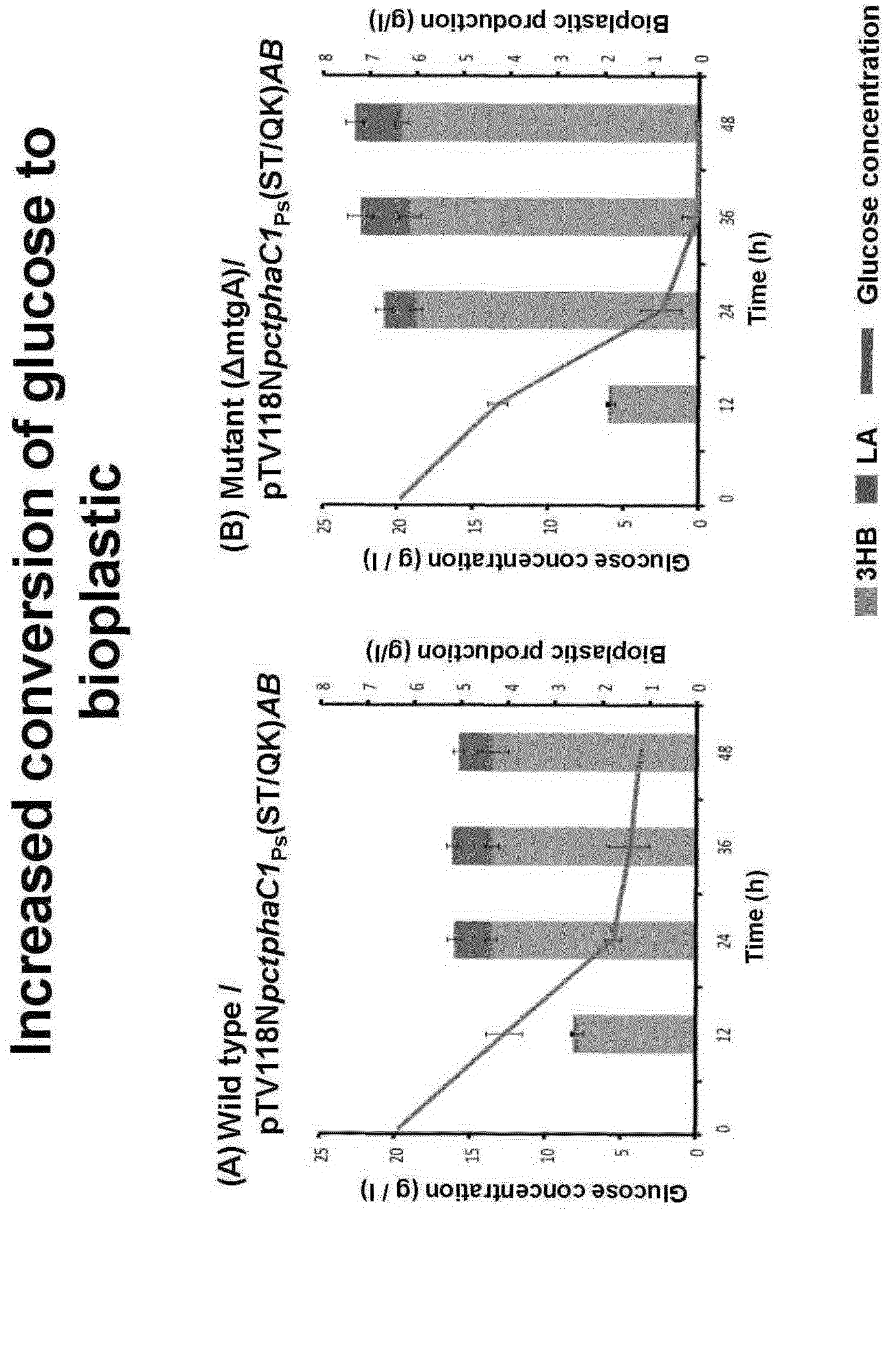
Increased conversion of glucose to bioplastic
(A) Wild type / pTV118NpctphaC1Ps(ST/QK)AB
(B) Mutant (ΔmtgA)/ pTV118NpctphaC1Ps(ST/QK)AB
Glucose concentration (g / l)
Biopastic production (g/l)
Time (h)
3HB
LA
Glucose concentration

[Fig. 3]

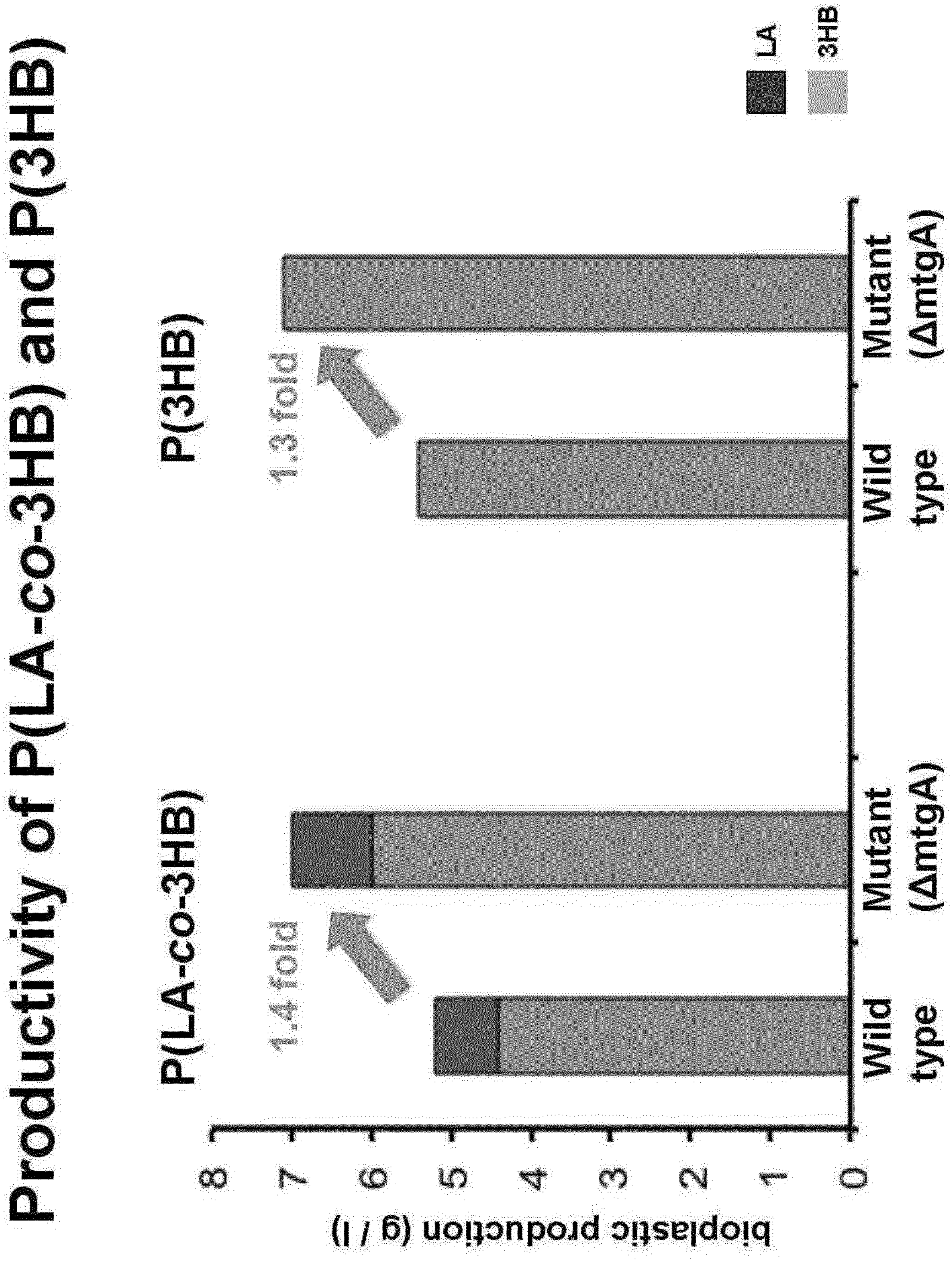
Productivity of P(LA-co-3HB) and P(3HB)
P(LA-co-3HB)
P(3HB)
1.4 fold
1.3 fold
LA
3HB
bioplastic production (g / l)
0
1
2
3
4
5
6
7
8
Wild type
Mutant (ΔmtgA)
Wild type
Mutant (ΔmtgA)

## INTERNATIONAL SEARCH REPORT

International application No. PCT/JP2016/065587

A. CLASSIFICATION OF SUBJECT MATTER
*C12N15/09*(2006.01)i, *C12N1/21*(2006.01)i, *C12P7/62*(2006.01)i

According to International Patent Classification (IPC) or to both national classification and IPC

B. FIELDS SEARCHED

Minimum documentation searched (classification system followed by classification symbols)
C12N15/09, C12N1/21, C12P7/62

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN), PubMed, CiNii, WPIDS/WPIX(STN)

C. DOCUMENTS CONSIDERED TO BE RELEVANT

| Category* | Citation of document, with indication, where appropriate, of the relevant passages | Relevant to claim No. |
|---|---|---|
| X<br>A | BABA, T., et al., Construction of Escherichia coli K-12 in-frame, signal-gene knockout mutants: the Keio collection, Molecular Systems Biology(2006), 2006.02.21, 2006. 0008, p. 1-11, Supplementary Table 2, refer to Supplementary Table 2 | 1,3,4,10,12<br>2,5-9,11 |
| A | Tsuyoshi UEHARA, "Cell wall shaping during bacterial morphogenesis", Journal of Japanese Biochemical Society, 25 May 2013 (25.05.2013), vol.85, no.5, pages 349 to 353 | 1-12 |
| P,X | Ryosuke KADOYA et al., "Nyusan Base Bio Plastic no Sosei to 'Fusen-gata' Biseibutsu Kojo", Convertech, 15 April 2016 (15.04.2016), vol.44, no.4, whole no.517, pages 125 to 127 | 1-12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August 2016 (10.08.16) | 30 August 2016 (30.08.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LE MEUR S. et al.** *Microb Cell Fact,* 2014, vol. 13, 131
- **MATSUMOTO K. et al.** *J Biotechnol,* 2011, vol. 156, 214-217
- **MATSUMOTO K. et al.** *Biomacromolecules,* 2013, vol. 14, 1913-1918
- **ELSDEN SR ; GILCHRIST FM ; LEWIS D ; VOLCANI BE.** Properties of a fatty acid forming organism isolated from the rumen of sheep. *J Bacteriol,* 1956, vol. 72, 681-689
- **MATSUSAKI H ; MANJI S ; TAGUCHI K ; KATO M ; FUKUI T ; DOI Y.** Cloning and molecular analysis of the Poly(3-hydroxybutyrate) and Poly(3-hydroxybutyrate-co-3-hydroxyalkanoate) biosynthesis genes in Pseudomonas sp. strain 61-3. *J Bacteriol,* 1998, vol. 180, 6459-6467
- **YAMADA M ; MATSUMOTO K ; SHIMIZU K ; URAMOTO S ; NAKAI T ; SHOZUI F et al.** Adjustable mutations in lactate (LA)-polymerizing enzyme for the microbial production of LA-based polyesters with tailor-made monomer composition. *Biomacromolecules,* 2010, vol. 11, 815-819
- **TAGUCHI S ; YAMADA M ; MATSUMOTO K ; TAJIMA K ; SATOH Y ; MUNEKATA M et al.** A microbial factory for lactate-based polyesters using a lactate-polymerizing enzyme. *Proc Natl Acad Sci U S A,* 2008, vol. 105, 17323-17327
- **SOM T ; TOMIZAWA J.** Origin of replication of Escherichia coli plasmid RSF 1030. *Mol Gen Genet,* 1982, vol. 187, 375-383
- **KADOYA R ; BAEK JH ; SARKER A ; CHATTORAJ DK.** Participation of chromosome segregation protein ParAI of Vibrio cholerae in chromosome replication. *J Bacteriol,* 2011, vol. 193, 1504-1514
- **YANISCH-PERRON C ; VIEIRA J ; MESSING J.** Improved M13 phage cloning vectors and host strains: nucleotide sequences of the M13mp18 and pUC19 vectors. *Gene,* 1985, vol. 33, 103-119
- **BABA T ; ARA T ; HASEGAWA M ; TAKAI Y ; OKUMURA Y ; BABA M et al.** Construction of Escherichia coli K-12 in-frame, single-gene knockout mutants: the Keio collection. *Mol Syst Biol,* 2006, vol. 2, 2006-0008
- **DE LORENZO V ; HERRERO M ; JAKUBZIK U ; TIMMIS KN.** Mini-Tn5 transposon derivatives for insertion mutagenesis, promoter probing, and chromosomal insertion of cloned DNA in gram-negative eubacteria. *J Bacteriol,* 1990, vol. 172, 6568-6572
- **NDUKO JM ; MATSUMOTO K ; OOI T ; TAGUCHI S.** Enhanced production of poly(lactate-co-3-hydroxybutyrate) from xylose in engineered Escherichia coli overexpressing a galactitol transporter. *Appl Microbiol Biotechnol,* 2014, vol. 98, 2453-2460
- **KICHISE T ; TAGUCHI S ; DOI Y.** Enhanced accumulation and changed monomer composition in polyhydroxyalkanoate (PHA) copolyester by in vitro evolution of Aeromonas caviae PHA synthase. *Appl Environ Microbiol,* 2002, vol. 68, 2411-2419
- **SPIEKERMANN P ; REHM BH ; KALSCHEUER R ; BAUMEISTER D ; STEINBUECHEL A.** A sensitive, viable-colony staining method using Nile red for direct screening of bacteria that accumulate polyhydroxyalkanoic acids and other lipid storage compounds. *Arch Microbiol,* 1999, vol. 171, 73-80
- **MATSUMOTO K ; OKEI T ; HONMA I ; OOI T ; AOKI H ; TAGUCHI S.** Efficient (R)-3-hydroxybutyrate production using acetyl CoA-regenerating pathway catalyzed by coenzyme A transferase. *Appl Microbiol Biotechnol,* 2013, vol. 97, 205-210
- **SAMBROOK J RD.** Molecular Cloning: A Laboratory Manual. Cold Spring Harbor Laboratory, 2001
- **WASHIO K ; LIM SP ; ROONGSAWANG N ; MORIKAWA M.** Identification and characterization of the genes responsible for the production of the cyclic lipopeptide arthrofactin by Pseudomonas sp. MIS38. *Biosci Biotechnol Biochem,* 2010, vol. 74, 992-999
- **NDUKO JM ; MATSUMOTO K ; OOI T ; TAGUCHI S.** Effectiveness of xylose utilization for high yield production of lactate-enriched P(lactate-co-3-hydroxybutyrate) using a lactate-overproducing strain of Escherichia coli and an evolved lactate-polymerizing enzyme. *Metab Eng,* 2013, vol. 15, 159-166
- **DATSENKO KA ; WANNER BL.** One-step inactivation of chromosomal genes in Escherichia coli K-12 using PCR products. *Proc Natl Acad Sci U S A,* 2000, vol. 97, 6640-6645
- **KITAGAWA M ; ARA T ; ARIFUZZAMAN M ; IOKA-NAKAMICHI T ; INAMOTO E ; TOYONAGA H et al.** Complete set of ORF clones of Escherichia coli ASKA library (a complete set of E. coli K-12 ORF archive): unique resources for biological research. *DNA Res,* 2005, vol. 12, 291-299